(19) Europäisches Patentamt
European Patent Office
Office européen des brevets

(11) Veröffentlichungsnummer: **0 131 779**
**A1**

(12) # EUROPÄISCHE PATENTANMELDUNG

(21) Anmeldenummer: 84107069.1

(51) Int. Cl.⁴: C 07 D 309/32

(22) Anmeldetag: 20.06.84

(30) Priorität: 15.07.83 DE 3325505
25.11.83 DE 3342598

(43) Veröffentlichungstag der Anmeldung:
23.01.85 Patentblatt 85/4

(84) Benannte Vertragsstaaten:
AT BE CH DE FR GB IT LI LU NL SE

(71) Anmelder: DYNAMIT NOBEL AKTIENGESELLSCHAFT
Postfach 1209
D-5210 Troisdorf, Bez. Köln(DE)

(72) Erfinder: Schmidt, Hans-Georg, Dr.
Porzer Strasse 15
D-5216 Niederkassel(DE)

(72) Erfinder: Steffen, Klaus-Dieter, Dr.
Röckelstrasse 36
D-5202 Hennef 1(DE)

(72) Erfinder: Meyer, Günther, Dr.
Augustinusstrasse 6
D-5210 Troisdorf(DE)

(54) **Verfahren zur Herstellung von 3,4-Dihydro-alpha-pyronen.**

(57) Die vorliegende Erfindung befaßt sich mit der Herstellung von, gegebenenfalls alkylsubstituierten, 3,4-Dihydro-α-pyronen durch Thermolyse der korrespondierenden 5-Hydroxy-methyl-2(3H) furanone. Es wurde gefunden, daß diese bekannte Thermolyse in Gegenwart einer großen Reihe von Katalysatoren bereits bei relativ niedriger Temperatur durchführbar ist. Die einsetzbaren Katalysatoren umfassen Oxide und Mischoxide von den Elementen der III und IV. Hauptgruppe und der IV. bis VII. Nebengruppe des Periodischen Systems der Elemente. Auch Zinkoxid und Mischoxide des Magnesiums lassen sich einsetzen. Weiterhin zählen zu den einsetzbaren Katalysatoren Schwefel- und Phosphorsäuren sowie deren organische Derivate. Auch Lewis-Säuren lassen sich als Katalysatoren einsetzen. Zu den katalytisch besonders wirksamen Mischoxiden des Aluminiums und Siliciums zählen hauptsächlich Schichtsilikate und Alumosilikate. In einer speziellen Durchführungsform wird die Reaktion in hochsiedenden Lösungsmitteln durchgeführt. Bei dieser Arbeitsweise wird das entstehende Dihydropyron zweckmäßigerweise sofort nach seiner Entstehung aus dem Reaktionsmedium zusammen mit dem Reaktionswasser abdestilliert.

EP 0 131 779 A1

Croydon Printing Company Ltd

Troisdorf, den 28.05.1984
OZ: 83036/068 Dr.Sk/Ce

DYNAMIT NOBEL AKTIENGESELLSCHAFT
Troisdorf, Bez. Köln

## Verfahren zur Herstellung von 3,4-Dihydro-α-pyronen

Gegenstand der vorliegenden Erfindung ist ein Verfahren zur Herstellung von 3,4-Dihydro-α-pyronen durch Erhitzen der entsprechenden 5-Hydroxymethylfuranone auf Temperaturen zwischen 200 und 500 °C.

In der US-PS 43 48 535 wird die Herstellung von 3,4-Dihydro-α-pyronen der Formel

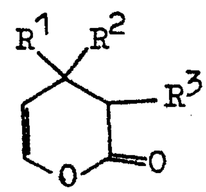

in der $R^1$, $R^2$ und $R^3$ für Wasserstoff und/oder Alkylreste mit 1 bis 10 C-Atomen steht, durch Erhitzen von Lactonen der Formel

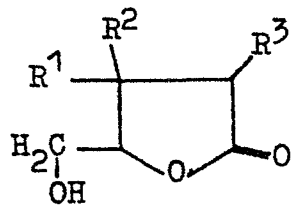

in der $R^1$, $R^2$ und $R^3$ die oben genannte Bedeutung haben, erwähnt. Bei dem dort beschriebenen Verfahren sind in dem angegebenen Temperaturbereich zwischen 200 und 500 °C die Reaktionsgeschwindigkeiten relativ gering.

Die Herstellung von 3,4-Dihydro-α-pyronen durch Thermolyse der genannten Lactone wird beispielsweise weiterhin in den DE-OS's 29 52 068 und 31 38 843 beschrieben. Bei den in diesen Schutzrechten abgehandelten Verfahren wird auch die Mitverwendung von Lösungsmitteln erwähnt; doch sieden die dort genannten Lösungsmittel alle unter 200 °C und dienen lediglich zum Auflösen der Einsatzverbindung (II) oder als gasförmiges Schleppmittel. Die Herstellung wird so beschrieben, daß man die Ausgangsverbindung durch ein erhitztes Rohr schickt, das mit silikatischen Materialien gefüllt ist. Bekanntlich ist aber eine genaue, in Längs- und Querrichtung konstante und einheitliche Temperaturführung bei solchen Reaktoren, in denen auch Wärmetönungen - endo- oder exothermer Art - auftreten, wegen schlechter Wärmeübergänge zumindest bei größeren Rohrdurchmessern äußerst schwierig.

Es bestand deshalb die Aufgabe, das in der DE-OS 29 52 068 genannte Verfahren derart zu verbessern, daß die Reaktionsgeschwindigkeiten der Umsetzung zu den gewünschten 3,4-Dihydro-α-pyronen erhöht und damit die Raum-Zeit-Ausbeute bei der Thermolyse des Hydroxymethylfuranons verbessert wird.

In Erfüllung dieser Aufgabe wurde nun ein Verfahren zur Herstellung von, gegebenenfalls in 3- oder 4-Stellung alkylsubstituierten 3,4-Dihydro-α-pyronen durch Erhitzen von 5-Hydroxymethyl-(3H)-furanonen auf Temperaturen zwischen 200 und 500 °C gefunden, das dadurch gekennzeichnet ist, daß das Erhitzen in Gegenwart der im Patentanspruch 1 genannten Katalysatoren erfolgt.

Bei Verwendung dieser Katalysatoren wird die Reaktionsgeschwindigkeit der Thermolyse erhöht und damit die Raum-Zeit-Ausbeute verbessert. Die erfindungsgemäß aufgefundenen Katalysatoren ermöglichen es, die Thermolyse im unteren Temperaturbereich dieses bekannten Verfahrens durchzuführen.

Als Katalysatoren eignen sich eine große Anzahl von Verbindungen: Alle beanspruchten Verbindungen üben einen mehr oder minder ausgeprägten katalytischen Einfluß auf die Thermolyse aus. Bei dem in der bereits genannten US-PS 43 48 535 erwähnten Verfahren ist als katalytisch wirksame Verbindung Montmorillonit anwesend. Es muß jedoch als überraschend angesehen werden, daß diese Reaktion durch eine weitere große Zahl von Verbindungen katalysiert wird.

Bei den katalytisch wirksamen Metalloxiden handelt es sich hauptsächlich um die Oxide und Mischoxide derjenigen Metalle, die in der III. und den höheren Gruppen des Periodischen Systems der Elemente stehen. Zusätzlich können noch Zinkoxid und Mischoxide des Magnesiums eingesetzt werden. Aus der III. Gruppe des Periodischen Systems eignen sich hauptsächlich die Oxide des Aluminiums und Galliums sowie diejenigen der Seltenen Erdmetalle. Vertreter von Metalloxiden von Elementen der IV. Gruppe des Periodischen Systems sind Siliciumdioxid sowie die Oxide von Titan, Zirkon, Hafnium und Thorium. Bei den Oxiden der Metalle der V. bis VII. Gruppe des Periodischen Systems handelt es sich um die Metalloxide von Elementen der entsprechenden Nebengruppen, wobei hauptsächlich die Elemente der 3. Periode in Form ihrer Oxide einsetzbar sind. Dies gilt auch für die Oxide der Metalle der VIII. Gruppe, von denen hauptsächlich die Oxide der Eisenmetalle katalytische Wirkung besitzen.

Aus dieser Aufzählung geht hervor, daß Oxide von Elementen

mit den Ordnungszahlen 13/14, 22 bis 28, 30/31, 39 bis 42, 57 bis 74 und 90 erfindungsgemäß als Katalysatoren eingesetzt werden können.

Die Metalloxide brauchen nicht in reiner Form vorzuliegen. Dies gilt besonders, wenn sie in Form von Mischoxiden verwendet werden. Unter Mischoxiden sollen besonders auch natürlich vorkommende Mineralien verstanden werden, die sich, besonders in aufgearbeiteter Form, ebenfalls als Katalysatoren erfindungsgemäß einsetzen lassen. Zu den Aufbereitungsmethoden zählen u.a. Flotation, Reinigung (z.B. mit Chlorwasserstoff), Erhitzen, Formgebungsverfahren und Verfahren zur Erhöhung der Porosität dieser Mineralien.

Mineralien und Mischoxide, die sich besonders gut erfindungsgemäß als Katalysatoren einsetzen lassen, sind Aluminiumsilikate und Schichtsilikate. Unter Aluminiumsilikaten sollen dabei hauptsächlich solche Silikate verstanden werden, in denen die Si-Atome z.T. durch Al-Atome im Gittergerüst ersetzt sind und die Restladungen durch K-, Na- oder Ca-Ionen ausgeglichen werden. Sie haben Porenweiten von 2 bis 20 Å und können natürlichen oder synthetischen Ursprungs sein. Beispiele für Aluminiumsilikate sind die Zeolithe und Montmorillonite, die auch als entsprechende synthetische Produkte eingesetzt werden können. Zu den Schichtsilikaten zählen z.B. Saponit, Beidellit, Hectorit, Fluorhectorit, Vermiculit, Pyrophyllit, Talkum, Muscovit, Phlogopit, Kaolinit, Illit oder Chlorit. Die katalytische Wirkung dieser Verbindungen hängt teilweise auch von der Porengröße oder ihrem Gehalt an Alkali- oder Erdalkalimetallen (z.B. bei den Zeolithen) ab.

Die Katalysatoren dieser Gruppe können in den üblichen Handelsformen wie Pulver, Kugeln, Ringe, Stäbchen, Sättel oder Tabletten eingesetzt werden. Es ist aber auch möglich,

sie auf ein geeignetes Trägermaterial aufzubringen und sie in dieser Form einzusetzen.

Unter den Lewis-Säuren sollen hauptsächlich Bortrifluorid, die Bromide von Phosphor und Aluminium und vor allem die Chloride von Bor, Aluminium, Phosphor, Antimon, Wismut, Eisen, Zink und Zinn verstanden werden. Als Brönstedsäuren eignen sich sowohl anorganische Säuren, wie z.B. $H_3PO_4$, $H_2SO_4$ als auch organische Säuren, wie z.B. p-Toluolsäure oder andere aromatische Sulfonsäuren.

Bevorzugte Ester sind Alkyl- und Arylester der Phosphorsäure wie z.B. Trimethylphosphat und Trikresylphosphat.

Bevorzugte Säureanhydride sind $P_2O_5$ und Phthalsäureanhydrid.

Die Durchführung der Reaktion mit Hilfe der beanspruchten Katalysatoren erfolgt bei den Feststoffkatalysatoren zweckmäßigerweise in einem beheizten Rohr, das mit dem Katalysatormaterial gefüllt ist. Das als Ausgangsprodukt eingesetzte Furanon läßt man in dieses Rohr eintropfen und das Umsetzungsprodukt dann in einer gekühlten Vorlage auffangen.

Es ist jedoch auch möglich, das Ausgangsprodukt zusammen mit dem Katalysator in einem Rührgefäß zu erhitzen und das entstehende Reaktionsprodukt - d.h. Wasser und das gewünschte Dihydropyron - über einen Destillationsaufsatz kontinuierlich abzunehmen. Diese Arbeitsweise empfiehlt sich besonders beim Einsatz von Katalysatoren aus der Gruppe der Schwefel- und Phosphorsäuren sowie von Lewis-Säuren. Dabei kann auch in Gegenwart von Lösungsmitteln gearbeitet werden. Es ist weiterhin auch möglich, diese Katalysatoren auf thermisch beständige Trägermaterialien aufzutragen und die Reaktion in einem Thermolyserohr mit Festbettkatalysator durchzuführen.

Als Lösungsmittel eignen sich sowohl aliphatische als auch aromatische Kohlenwasserstoffe, deren Siedepunkte möglichst über 100 °C liegen sollen. Das Lösungsmittel kann bei der Reaktionstemperatur gasförmig oder flüssig vorliegen, so daß auch Lösungsmittel einsetzbar sind, deren Siedepunkte unterhalb der Reaktionstemperatur liegen. In einem solchen Fall kann dabei unter Druck gefahren werden und das gewünschte α-Pyron sowie das bei der Thermolyse entstehende Wasser über ein Druckhalteventil herausdestilliert werden. Als Beispiele für Lösungsmittel seien Dekalin, Toluol oder Xylol genannt. Auch flüssige Phosphorsäureester lassen sich einsetzen, die dann zusätzlich noch den Vorteil haben, daß sie gleichzeitig katalytisch wirksam sind.

Eine bevorzugte Durchführungsform ergibt sich beim Einsatz von flüssigen Lösungsmitteln mit Siedepunkten über 100 °C, bevorzugt über 200 °C. Bei einer solchen Durchführungsform werden als Katalysatoren bevorzugt Alumosilikate eingesetzt.

Die bei der bevorzugten Durchführungsform einsetzbaren Lösungsmittel sind global den Wärmeträgerölen zuzuordnen. Diese können sowohl natürlichen als auch synthetischen Ursprungs sein. So eignen sich beispielsweise Mineralölraffinate, Paraffine, polycyclische Kohlenwasserstoffe oder auch Mehrkernaromaten. Die Mehrkernaromaten können entweder direkt oder über Gruppierungen aus der Gruppe $-CH_2-$, $-O-$, $-SO_2-$, $-OSiR_2O-$ (R=Alkylen) miteinander verbunden sein. Auch entsprechende partiell hydrierte oder durch Chlor substituierte Mehrkernaromaten können eingesetzt werden. In der Regel werden zur Herabsetzung des Schmelzbereiches Isomerenmischungen oder Gemische unterschiedlicher chemischer Struktur verwendet.

In diesen Lösungsmitteln wird der Katalysator unter Rühren

suspendiert. Als Katalysator eignen sich besonders die oben genannten Alumosilikate mit beliebiger geometrischer Form, z.B. als Kügelchen, Stäbchen oder auch in Pulverform, in denen die Si-Atome z.T. durch Al-Atome im Gittergerüst ersetzt sind und die Restladungen durch K-, Na- oder Ca-Ionen ausgeglichen werden. Sie haben Porenweiten von 2 bis 20 Å und können natürlichen oder synthetischen Ursprungs sein.

Der Katalysator muß weiterhin so gewählt werden, daß er das hochsiedende Lösungsmittel nicht zersetzt. So eignen sich z.B. beim Einsatz von mehrkernigen Aromaten, die über $-CH_2$-Brücken miteinander verknüpft sind, Zeolithe besonders gut als Katalysatoren, während Montmorillonite in diesem Fall zersetzend wirken. Es ist also immer in Vorversuchen abzuklären, ob das jeweils einzusetzende Alumosilikat mit dem Lösungsmittel bei den beanspruchten Temperaturen verträglich ist.

Besonders hohe Ausbeuten an dem Endprodukt beim Arbeiten in hochsiedenden Lösungsmitteln werden nur im Temperaturbereich zwischen 300 und 400 °C erhalten. Bei niederen Temperaturen sind die Ausbeuten erheblich geringer, während das Arbeiten bei höheren Temperaturen zu mehr Zersetzungsprodukten führt.

Das Gewichtsverhältnis von Lösungsmittel zu Alumosilikat kann in den Grenzen von 2:(0,2 bis 2,0), vorzugsweise aber von 2:(0,8 bis 1,0), gewählt werden.

Bei der praktischen Durchführung der Verfahrensweise des Arbeitens in hochsiedenden Lösungsmitteln wird zu der auf die gewünschte Reaktionstemperatur erhitzten Lösungsmittel/Katalysatormischung das Furanon (II) in aufgeschmolzener Form zudosiert - in Portionen oder kontinuierlich - und

das Zielprodukt (I) nach seiner Bildung möglichst sofort mit dem Wasser abdestilliert. Um beim Abdestillieren des Zielproduktes zusammen mit dem Wasser aus dem Reaktionsmedium das Ausgangsprodukt und Lösungsmittel nicht mitzureißen, wird zweckmäßigerweise, in an sich bekannter Weise, ein Dephlegmator zwischengeschaltet, der auf der Siedetemperatur des Endproduktes (I) gehalten wird.

Während der Reaktion wird ausreichend gerührt, um gute Wärmeübergänge und guten Stoffaustausch zu gewährleisten. Es kann mit einem schwachen Stickstoffstrom inertisiert werden.

Die Trennung des aus dem Reaktionsmedium abdestillierten Endprodukts von dem bei der Reaktion gebildeten, ebenfalls mitabdestillierten Wasser erfolgt auf an sich bekannte Weise. Im allgemeinen kann aufgrund der Unlöslichkeit der Pyrone im Wasser eine mechanische Trennung der beiden Komponenten erfolgen. Anschließend wird das 3,4-Dihydro-$\alpha$-pyron fraktioniert destilliert. Es werden Ausbeuten um 70 % der Theorie, bezogen auf eingesetztes 5-Hydroxymethyl-$\gamma$-butyrolacton, erzielt.

Pro Katalysatormengeneinheit kann das Vielfache der Furanon-(II)-Menge eingesetzt werden. Auch bei Schwarzfärbung der Katalysatormischung ist diese noch voll reaktionsfähig; ihre Verwendbarkeit wird nur durch allmähliche Viskositätserhöhung und daraus folgende Rührunfähigkeit begrenzt.

Nach beendeter Reaktion kann der Katalysator regeneriert werden. Dies erfolgt z.B. durch Abfiltrieren, Auswaschen mit einfachen Lösungsmitteln (z.B. Aceton) und anschließendem Glühen; daraufhin ist der Katalysator wieder voll einsatzfähig. Aus Kostengründen empfiehlt sich aber auch eine einfache Deponierung oder Vernichtung des Katalysator/Lösungsmittel-Endsumpfes.

Die erfindungsgemäß herstellbaren 3,4-Dihydro-α-pyrone sind wertvolle Zwischenprodukte zur Herstellung von Cyclopropancarbonsäureestern, die wiederum als Ausgangsprodukte für synthetische insektizid wirkende Pyrethroide eingesetzt werden.

Beispiele 1 bis 12

Ein elektrisch beheiztes, senkrecht stehendes Thermolyserohr von 1,5 cm Länge und einem Durchmesser von 1,24 cm wird mit 1,0 g der in der Tabelle 1 genannten Katalysatoren in Kugelform gefüllt. 2 g Dihydro-5-hydroxymethyl-4,4-dimethyl-2(3H)furanon werden auf die Katalysatorfüllung unter $N_2$ aufgetropft. Das Reaktionsgemisch wird in einer gekühlten Vorlage aufgefangen und gaschromatographisch analysiert. Die Ergebnisse sind in Tabelle 1 aufgeführt. Die Prozentangaben in den Spalten 3 und 5 beziehen sich jeweils auf eingesetztes Ausgangsprodukt.

Die in den Beispielen 7 und 8 genannten Montmorillonite sind Handelsprodukte, die sich aufgrund unterschiedlicher Behandlung durch ihre Oberfläche und das Porenvolumen unterscheiden.

Tabelle 1

| Beispiel Nr. | Katalysator Kugelform | nach der Thermolyse in der Vorlage aufgefangenes Produkt (%) | Temp. °C | Ausbeute an 4,4-Dimethyl-3,4-dihydro-α-pyron (%) |
|---|---|---|---|---|
| 1 | kein | 96 | 330 | 0 |
| 2 | Zeolith Mordenit Na-Form | 96 | 330 | 48 |
| 3 | Mordenit H-Form | 88 | 330 | 76 |

Fortsetzung Tabelle 1

| Beispiel Nr. | Katalysator Kugelform | nach der Thermolyse in der Vorlage aufgefangenes Produkt (%) | Temp. °C | Ausbeute an 4,4-Dimethyl-3,4-dihydro-α-pyron (%) |
|---|---|---|---|---|
| | Synthetische Zeolithe | | | |
| 4 | A-Typ | 91 | 330 | 30 |
| 5 | Z-Typ | 85 | 330 | 70 |
| 6 | A-Typ in Ca-Form | 95 | 330 | 10 |
| 7 | Montmorillonit KA 2 | 93 | 330 | 32 |
| 8 | Montmorillonit K 306 | 90 | 330 | 50 |
| 9 | $SiO_2$ | 90 | 330 | 51 |
| 10 | $Al_2O_3$ | 87 | 330 | 69 |
| 11 | $Al_2O_3/SiO_2$ mit $H_3PO_4$ | 92 | 330 | 65 |
| 12 | Tonerde | 80 | 360 | 62 |

Beispiele 13 bis 19

In der gleichen Apparatur wie bei den Beispielen 1 bis 12 wurden die in der Tabelle 2 genannten Katalysatoren getestet. Die Katalysatoren lagen in Pulverform vor. Die Versuchsdurchführung erfolgte auf die gleiche Weise wie bei den Beispielen 1 bis 12. Die Ergebnisse sind in Tabelle 2 zusammengefaßt.

Tabelle 2

| Beispiel Nr. | Katalysator | nach der Thermolyse in der Vorlage aufgefangenes Produkt (%) | Temp. °C | Ausbeute an 4,4-Dimethyl-3,4-dihydro-α-pyron (%) |
|---|---|---|---|---|
| 13 | Zeolith | ca. 90 | 350 | 28 |
| 14 | $Al_2O_3$ | ca. 90 | 350 | 17 |

| | | | | |
|---|---|---|---|---|
| 15 | $ZrO_2$ | 90 | 350 | 15 |
| 16 | $V_2O_5$ | 38 | 350 | 37 |
| 17 | $ThO_2$ auf Träger | 85 | 350 | 28 |
| 18 | $TiO_2$ auf Träger | 90 | 350 | 18 |
| 19 | Montmorillonit | 90 | 330 | 24 |

Beispiele 20 bis 31

In einem Rührkolben wird Dihydro-5-hydroxy-methyl-4,4-dimethyl-2(3H)furanon mit Katalysator und/oder Lösungsmittel vorgelegt und auf die in Tabelle 3 genannten Temperaturen erhitzt. Die entstehenden Destillationsprodukte werden sofort destillativ abgenommen. Die Ergebnisse gehen aus Tabelle 3 hervor. Die in der letzten Spalte genannten Ausbeuten beziehen sich auf eingesetztes Ausgangsprodukt.

Tabelle 3

| Beispiel Nr. | Katalysator | Lösungsmittel | Temp. °C | Ausbeute an 4,4-Dimethyl-3,4-dihydro-α-pyron (%) |
|---|---|---|---|---|
| 20 | $Al_2O_3$ Kugeln | - | 330 | 26 |
| 21 | $SiO_2$ Kugeln | - | 330 | 29 |
| 22 | Zeolith (Pulver) | - | 330 | 28 |
| 23 | Zeolith | Trikresylphosphat | 330 | 52 |
| 24 | $H_2SO_4$ | | | |
| 25 | Polyphosphorsäure | Dekalin | 200 | 10 |
| 26 | $P_2O_5$ | | 320 | 37 |
| 27 | p-Toluolsulfonsäure | - | 250 | 15 |
| 28 | $K_2HPO_4$ | | 350 | 10 |
| 29 | $KHSO_4$ | | 350 | 30 |
| 30 | Kein | Trikresylphosphat | 330 | 8 |
| 31 | Kein | Kein | 350 | 0 |

Beispiel 32

In einen 1-Liter-Vierhalskolben, versehen mit Rührer, Thermometer, beheizbarem Tropftrichter und Destillationsaufsatz werden 200 g eines Isomerengemisches mehrkerniger Aromaten, die über $-CH_2$-Brücken miteinander verknüpft sind (im Handel unter der Bezeichnung "Marlotherm S" erhältlich) und 90 g eines Zeoliths in Kugelform mit 5 Å Porenweite eingefüllt und auf 350 °C aufgeheizt. Der untere Teil des Destillationsaufsatzes bestand aus einer auf ca. 220 °C aufgeheizten Vigreux-Kolonne. Aus dem beheizten Tropftrichter wurden innerhalb von 30 Minuten 60 g 4,4-Dimethyl-5-hydroxymethyl-γ-butyrolacton (OH-V) zugetropft und 30 Minuten nachreagieren gelassen. Dabei destillierten über den Destillationsaufsatz das endstandene 3,3-Dihydro-4,4-dimethyl-α-pyron (DDP) und das Kondensationswasser ab. Diese Portionierung des OH-V wurde noch 30 x wiederholt, so daß insgesamt 1,8 kg der Ausgangssubstanz durchgesetzt wurden.

Das abdestillierte DDP wurde zusammen mit dem abdestillierten Wasser kondensiert, die Wasserphase abgeschieden und die organische Phase über eine Kolonne aufdestilliert (Kp.: 69 °C/14 Torr). Es wurden 1150 g 3,4-Dihydro-4,4-dimethyl-α-pyron (DDP) mit einer Reinheit von 97 % erhalten, was einer Ausbeute von 70,8 % der Theorie, bezogen auf eingesetztes OH-V, entspricht. Wird die nicht umgesetzte, zurückgewonnene OH-V-Menge berücksichtigt, so ergibt sich eine Ausbeute von 73,1 % der Theorie.

Beispiel 33

Der im Beispiel 32 verwendete Katalysator wurde über eine grobe Fritte abfiltriert, mit Aceton gut gewaschen und über Nacht bei 500 °C geglüht. Das Beispiel 32 wurde daraufhin in seinen Bedingungen mit dem auf diese Weise regenerierten Katalysator wiederholt. Es wurden jedoch nur

1,2 kg 4,4-Dimethyl-5-hydroxymethyl-γ-butyrolacton durchgesetzt.

Es wurden 807 g 3,4-Dihydro-4,4-dimethyl-α-pyron mit einer Reinheit von 98 % erhalten. Ausbeute: 75,3 % der Theorie, bezogen auf eingesetztes OH-V; nach Abzug von zurückgewonnenem Ausgangsprodukt liegt die Ausbeute bei 78,2 % der Theorie.

Beispiel 34

In der Apparatur entsprechend Beispiel 32 wurden 200 g des in Beispiel 32 genannten Isomerengemischs und 90 g eines Zeoliths mit 3 Å Porenweite auf 350 °C erhitzt und in Portionen von jeweils 60 g insgesamt 1,8 kg 4,4-Dimethyl-hydroxymethyl-γ-valerolacton durchgesetzt. Nach Abscheidung des Reaktionswassers wurde die organische Phase fraktioniert. Man erhielt 1091 g DDP mit einer Reinheit von 97 %. Ausbeute: 67.2 % der Theorie, nach Abzug von rückgewonnenem OH-V: 70,0 % der Theorie.

Beispiel 35

Es wurde eine Apparatur aufgebaut, die aus einem 6-Liter-Mehrhalskolben mit Rührer, Thermometer, auf ca. 220 °C temperiertem Dephlegmator und Destillationsaufsatz sowie Tauchrohr bestand und über eine Dosierpumpe aus einer beheizten Vorlage kontinuierlich mit Produkt versorgt werden konnte. Die Apparatur wurde mit einem schwachen Stickstoffstrom inertisiert. In den Kolben wurden 1,2 kg des in Beispiel 32 genannten Isomerengemischs und 540 g eines Zeoliths mit 5 Å Porenweite gefüllt und auf 350 °C aufgeheizt. Aus der beheizten Vorlage wurden dann mit ca. 350 g/h insgesamt 23,2 kg 4,4-Dimethyl-5-hydroxymethyl-γ-butyrolacton kontinuierlich zudosiert und DDP mit dem Reaktionswasser entsprechend abdestilliert.

Danach wurde das Reaktionswasser abgeschieden und die

organische Phase aufdestilliert.
Ausbeute: 13,3 kg DDP mit einer Reinheit von 97 %, entsprechend 63,6 % der Theorie.

Beispiel 36

In die Laborapparatur entsprechend Beispiel 32 wurden 200 g des dort genannten Isomerengemischs und 90 g eines Zeoliths in Form von Kügelchen von 1 mm Durchmesser und einer Porenweite von 5 Å eingefüllt und auf 350 °C aufgeheizt. In Abständen von einer Stunde wurden in 60 g Portionen insgesamt 6,0 kg 4,4-Dimethyl-5-hydroxymethyl-γ-butyrolacton zudosiert. Es wurde entsprechend Beispiel 32 aufgearbeitet und das DDP isoliert.
Ausbeute: 3742,5 g DDP mit einer Reinheit von 97,5 % entsprechend 69,5 % der Theorie.

Beispiel 37

Dieser Versuch wurde wie in Beispiel 36 durchgeführt mit der Ausnahme, daß die Reaktion bei 370 °C durchgeführt und nach Zugabe von 1,2 kg OH-V abgebrochen wurde.
Ausbeute: 718 g DDP, Reinheit: 98 %, entsprechend 67,0 % der Theorie.

Beispiel 38

In einem 120-Liter-Edelstahl-Rührwerk (Tauchrohr, Dephlegmator, Kondensator mit Vorlagen) wurden 35 kg des in Beispiel 32 beschriebenen Lösungsmittels und 15 kg eines Ca-Na-Alumosilikates vorgelegt und auf 340 bis 350 °C aufgeheizt. Aus einer beheizten Vorlage wurden mit einer Geschwindigkeit von anfangs 5 kg/h, später 8 kg/h insgesamt 205,5 kg 4,4-Dimethylhydroxy-γ-valerolacton mittels einer Dosierpumpe kontinuierlich über das Tauchrohr zudosiert. Entsprechend der Zudosierung destillierten das Reaktionswasser und 3,4-Dihydro-4,4-dimethyl-α-pyron ab. Das Wasser wurde abgeschieden und die organische Phase in

einer größeren Laborkolonne aufdestilliert. Ausbeute: 130,6 kg mit einer Reinheit von 95 %, entsprechend 69,2 % der Theorie, bezogen auf eingesetztes Valerolacton. Insbegesamt wurden ca.2kg Valerolacton zurückgewonnen, die einem folgenden Ansatz zugeführt wurden.

Troisdorf, den 28.05.1984
OZ: 83036 /068 Dr.Sk/Ce

Patentansprüche:

1. Verfahren zur Herstellung von 3,4-Dihydro-α-pyronen der Formel (I)

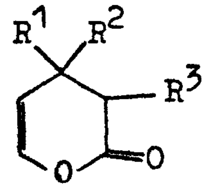

in der $R^1$, $R^2$ und $R^3$ für Wasserstoff und/oder gleiche oder ungleiche Alkylreste mit 1 bis 10 C-Atomen steht, durch Erhitzen von Furanonen der Formel (II)

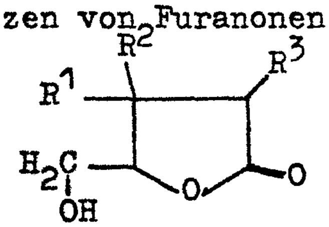

in der $R^1$, $R^2$ und $R^3$ die oben genannte Bedeutung haben, auf Temperaturen zwischen 200 und 500 °C, d a d u r c h g e k e n n z e i c h n e t , daß das Erhitzen in Gegenwart von Katalysatoren aus der Gruppe der

a) Oxide der Metalle der IV. bis VII. Nebengruppe des Periodischen Systems der Elemente und von Al, Si, Zn, Ga und von den Metallen der Eisengruppe, sowie entsprechender Mischoxide,

b) Lewis-Säuren und Brönstedsäuren,

c) Alkali- und Erdalkalisalze der Schwefel und Phosphorsäure,

d) Anhydride von anorganischen und organischen Säuren und

e) Ester anorganischer und organischer Säuren.

2. Verfahren gemäß Anspruch 1, dadurch gekennzeichnet, daß als Mischoxide des Aluminiums und/oder Siliciums Alumosilikate oder Schichtsilikate als Katalysatoren eingesetzt werden.

3. Verfahren zur Herstellung von 3,4-Dihydro-α-pyronen gemäß Anspruch 1 und 2, dadurch gekennzeichnet, daß man die Furanone im flüssigen Medium auf Temperaturen zwischen 300 und 400 °C in Gegenwart von Alumosilikaten erhitzt.

4. Verfahren gemäß Anspruch 3, dadurch gekennzeichnet, daß man das entstehende 3,4-Dihydro-α-pyron direkt anschließend an seine Bildung aus dem Reaktionsmedium abdestilliert und das Furanon in dem Maße zudosiert, wie das Pyron abdestilliert wird.

5. Verfahren gemäß Anspruch 3 oder 4, dadurch gekennzeichnet, daß man als flüssiges Medium Wärmeträgeröle mit Siedepunkten über 200 °C einsetzt.

Europäisches Patentamt

**EUROPÄISCHER RECHERCHENBERICHT**

0131779

Nummer der Anmeldung

EP 84107069.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | DE - A1 - 3 045 555 (DYNAMIT NOBEL)<br>* Anspruch 1; Seite 4, Zeilen 16-23; Beispiel 3 *<br>-- | 1-3 | C 07 D 309/32 |
| D,X | US - A - 4 348 535 (SCHMIDT)<br>* Beispiel 8 *<br>-- | 1-3 | |
| D,X | DE - A1 - 2 952 068 (DYNAMIT NOBEL)<br>* Beispiel 5 *<br>-- | 1,3 | |
| D,X | DE - A1 - 3 138 843 (DYNAMIT NOBEL)<br>* Ansprüche 1-5; Beispiele 1,3 *<br>---- | 1-3 | |

| RECHERCHIERTE SACHGEBIETE (Int Cl 4) |
|---|
| C 07 D 309/00 |

Der vorliegende Recherchenbericht wurde für alle Patentansprüche erstellt

| Recherchenort | Abschlußdatum der Recherche | Prüfer |
|---|---|---|
| WIEN | 29-09-1984 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82